# EUROPEAN PATENT SPECIFICATION

(11) **EP 2 564 874 B1**
(45) Date of publication and mention of the grant of the patent: **14.08.2019**
(21) Application number: 11774359.1
(22) Date of filing: 22.04.2011
(51) Int. Cl.: A61K 31/704, A61P 35/00, A61K 47/61, A61K 9/19, A61K 9/00, A61K 47/32

(54) **LYOPHILIZED FORMULATION OF PECTIN-ADRIAMYCIN CONJUGATE AND PREPARATION METHOD THEREOF**
LYOPHILISIERTE FORMULIERUNG AUS EINEM PECTIN-ADRIAMYCIN-KONJUGAT UND HERSTELLUNGSVERFAHREN DAFÜR
FORMULATION LYOPHILISÉE D'UN CONJUGUÉ DE PECTINE ET D'ADRIAMYCINE ET MÉTHODE DE PRÉPARATION DUDIT CONJUGUÉ

(30) Priority: 03.06.2010 CN 201010189969; 27.04.2010 CN 201010157856
(43) Date of publication of application: 06.03.2013
(73) Proprietor: Chongqing Lummy Pharmaceutical Co., Ltd., Chongqing 401123 (CN)
(72) Inventor: TANG, Xiaohai, Chongqing 401123 (CN); QIU, Yu, Chongqing 401123 (CN); PENG, Lilin, Chongqing 401123 (CN)
(74) Representative: Huang, Liwei
(86) International application number: PCT/CN2011/073153
(87) International publication number: WO 2011/134368

(56) References cited:
- CN-A- 101 134 109
- CN-A- 101 433 723
- CN-A- 101 721 714
- TANG XIAO-HAI ET AL: "Synthesis, characterization, and in vitro and in vivo evaluation of a novel pectin-adriamycin conju", BIOORGANIC & MEDICINAL CHEMISTRY, vol. 18, no. 4, 7 January 2010 (2010-01-07), pages 1599-1609, XP028649829, ISSN: 0968-0896, DOI: 10.1016/J.BMC.2009.12.076
- BAHETI A ET AL: "Excipients used in lyophilization of small molecules", JOURNAL OF EXCIPIENTS AND FOOD CHEMICALS, INTERNATIONAL PHARMACEUTICAL EXCIPIENTS COUNCIL, AMERICAS, UNITED STATES, vol. 1, no. 1, 1 June 2010 (2010-06-01), pages 41-54, XP002667508, ISSN: 2150-2668

## Description

### Field of the Invention

The invention belongs to the pharmaceutical field, and particularly relates to a lyophilized formulation of pectin-adriamycin conjugate and a preparation method thereof.

### Description of the Related Art

The Chinese patent application titled "Passive solid tumor-targeted anticancer prodrug and preparation method thereof' with application number 200910311854.0 discloses a passive solid tumor-targeted anticancer prodrug formed by bonding pectin and adriamycin, the preparation method is characterized by reacting small molecular pectin with Mw (Mw is weight-average molecular weight, and one of molecular weight representation methods of high molecular compounds) of 5,000 - 45,000 with adriamycin to obtain a pectin-adriamycin conjugate with Mw of 100,000 - 1,000,000, preparing a suspension, and treating the suspension using an ultra-high pressure nano homogenizer to obtain the passive solid tumor-targeted anticancer prodrug with particle size of 100nm - 200nm and melting point of 220 - 245°C; in which the pectin and the adriamycin are linked by an amido bond, and the pectin and the pectin are linked by an ester bond formed by condensing carboxyl groups and hydroxyl groups of pectin molecules. Specifically, the pectin-adriamycin conjugate is a macromolecular prodrug with thousands of molecular weight coupled by reacting carboxyl groups (-COOH) of pectin molecules with amino groups (-NH₂) of adriamycin to form an amido bond when pectin reacts with adriamycin (ADM) in the presence of dehydrant EDC·HCl, i.e. 1-(3-dimethylaminopropyl)-3-ethylcarbodiimide hydrochloride.

In the patent, the pectin-adriamycin conjugate is called pectin-adriamycin conjugate (hereinafter referred to as PAC), and the inventor found that solubility of the PAC is poor in application of the PAC, and repeated solubility tests showed that the PAC is insoluble. Insoluble drugs have two major problems in clinical application, one problem is low bioavailability due to low solubility of the drugs, and the other problem is that conventional formulation methods have certain restriction on application of the insoluble drugs.

### Summary of the Invention

With regard to insoluble drugs at present, a new formulation form (nanosuspension) has attracted more and more attention. Compared with conventional formulations, the nanosuspension has lots of advantages, for example, water is generally used as a dispersion system in a suspension, and the content of main drugs is high, thus being capable of avoiding adverse reactions resulting from some non-aqueous solvents and making administration by injection possible. According to Ostwald-Freundlich equation (lnC₂/C₁ = 2σM/RTρ(1/r²-1/r¹), r is particle size, and c is solubility), the particle size of drugs in the suspension is small and specific surface area is large, thus being capable of increasing solubility and bioavailability of the drugs to a certain extent. In addition, if the particle size of drugs is controlled below 200nm, nano particles can realize targeting in a better manner by virtue of EPR effect. In order to overcome insolubility defect of the PAC, the inventor prepared the PAC into a nanosuspension, and tried to solve the technical problem of insolubility by the technical means so as to achieve the purposes of improving bioavailability and facilitating preparation. Specifically, a method for preparing the insoluble pectin-adriamycin conjugate into a suspension comprises adding 500 - 900mg pectin-adriamycin conjugate and a stabilizer to 100mL sterile water for injection to obtain a mixture, grinding the mixture into a suspension, and then preparing the suspension into a nanosuspension.

However, the inventor found that particles of the prepared nanosuspension easily aggregated due to higher surface free energy, although two stabilizers (e.g. PVP K-30 and poloxamer 188) were added to a formula, long-term stability of the suspension was unsatisfactory. As particle size of nano particles is unstable, the PAC nanosuspension can not be stored for a long time in a suspension state, thus affecting clinical application effect of the formulation.

In order to overcome instability defect of the PAC nanosuspension, and ensure long-term stability thereof, the inventor provides a lyophilized formulation as described in claim 1, as well as advantageous embodiments as described in claims 2 to 3.

Futhermore, the present invention provides also a preparation method as described in claim 4, as well as advantageous embodiments as described in claims 5 to 13.

The preferable prefreezing condition is prefreezing at -40°C for 6h, and the lyophilizer is used for drying for at least 24h preferably.

Preferably, the method for preparing the insoluble pectin-adriamycin conjugate into the suspension comprises adding 500 - 900mg pectin-adriamycin conjugate and a stabilizer to 100mL sterile water for injection to obtain a mixture, and grinding the mixture into a suspension. In order to improve bioavailability of the ground suspension, the suspension can be further prepared into a nanosuspension, and preparation of the suspension into the nanosuspension in a high-pressure homogenizer is an effective, conventional, common and convenient treatment mode at present.

The stabilizer used is at least one of PVP, poloxamer, sodium dodecyl sulfate, polysorbate or hydroxypropyl methyl cellulose.

PVP is polyvinylpyrrolidone used as a stabilizer and a hydrophilic adjuvant, and preferably PVP K-30.

Poloxamer (used as a stabilizer and a hydrophilic adjuvant) can be of poloxamer 188 or poloxamer 407, and preferably poloxamer 188.

Sodium dodecyl sulfate (SDS) is an ionic surfactant used as a stabilizer, and can provide charge stabilization effect.

Polysorbate is a nonionic surfactant used as a stabilizer, and preferably polysorbate-80 (Tween 80).

Hydroxypropyl methyl cellulose (HPMC) is a high molecular polymer used as a stabilizer.

Stabilizers for preparing the suspension or nanosuspension can be of the surfactants or the high molecular polymer, can realize stable charge for stabilization.

Specifically, the lyophilized formulation of the invention is prepared by adding 500 - 900mg PAC, 3000 - 6000mg PVP K-30, 500 - 900mg poloxamer 188 and 4000 - 12000mg sucrose to 100mL sterile water for injection before lyophilization. The preferable formula is:
PAC (main drug): 776mg (drug loading rate is 25.8% converted based on adriamycin equivalent of 2mg/mL);
PVP K-30 (PVP is polyvinylpyrrolidone used as a stabilizer and hydrophilic adjuvant, K-30 is classification by molecular weight): 4000mg (w/v, 4%);
Poloxamer 188 (188 is model, acting as a stabilizer and hydrophilic adjuvant): 700mg (w/v, 0.7%);
Sucrose (lyophilized excipient): 80mg/mL (equivalent to 8000mg sucrose in each 100mL);
Sterile water for injection (solvent): 100mL.

Equivalently, the lyophilized formulation of the invention is prepared by adding 776mg PAC, 4000mg PVP K-30, 700mg poloxamer 188 and 8000mg sucrose to 100mL sterile water for injection before lyophilization.

Specifically, the preparation process comprises adding 40 - 120mg/mL (preferably 80mg/mL) lyophilized support agent (sucrose) to the PAC nanosuspension for full ultrasonic dissolution. In order to ensure proper lyophilization of the product, liquid height shall be generally controlled within 2cm.

A preparation method in the laboratory comprises the following steps: separately filling the PAC nanosuspension to be lyophilized (2mL/bottle) in 10mL penicillin bottles, placing the penicillin bottles capped with T shape stoppers (gap shall be reserved to allow moisture to spread out during lyophilization) in a refrigerator at -40°C - -80°C (preferably -40°C) for prefreezing for 4 - 8h (preferably 6h), and then placing in a lyophilizer for lyophilization for at least 24h.

The lyophilized product prepared by the method is characterized by reddish orange pie appearance, loose texture, saturated appearance without collapse and good resolubility, complete redissolution after adding formulated amount of sterile water for injection and gently shaking, and substantially no change in property and particle size after redissolution and before lyophilization, with average particle size after redissolution measured with a Malvern laser nano particle size analyzer below 200nm, and PDI value less than 0.25.

### Brief Description of the Drawings

Figure 1 is particle shape of the PAC nanosuspension.
Figure 2 is particle size of the PAC nanosuspension half a year later.
Figure 3 is particle size of the PAC nanosuspension. In the suspension, the average particle size of nano particles is 167.3nm, particle size distribution range is narrow, and PDI value is only 0.141.

### Description of the Preferred Embodiment

The lyophilized formulation provided in the invention is prepared by preparing an insoluble pectin-adriamycin conjugate into a suspension or nanosuspension, and adding a lyophilized support agent to the suspension for lyophilization treatment.

The method for preparing the insoluble pectin-adriamycin conjugate into the suspension or nanosuspension comprises adding 500 - 900mg pectin-adriamycin conjugate and a stabilizer to 100mL sterile water for injection to obtain a mixture, and grinding the mixture into the suspension.

The suspension can be further prepared into a nanosuspension. Specifically, a high-pressure homogenizer is used for preparing the suspension into the nanosuspension.

The stabilizer used is at least one of PVP, poloxamer, sodium dodecyl sulfate, polysorbate or hydroxypropyl methyl cellulose.

PVP is polyvinylpyrrolidone used as a stabilizer and a hydrophilic adjuvant, and preferably PVP K-30.

Poloxamer (used as a stabilizer and a hydrophilic adjuvant) can be of poloxamer 188 or poloxamer 407, and preferably poloxamer 188.

Sodium dodecyl sulfate (SDS) is an ionic surfactant used as a stabilizer, and can provide charge stabilization effect.

Polysorbate is a nonionic surfactant used as a stabilizer, and preferably polysorbate-80 (Tween 80).

Hydroxypropyl methyl cellulose (HPMC) is a high molecular polymer used as a stabilizer.

Stabilizers for preparing the suspension or nanosuspension can be of the surfactants or the high molecular polymer, and can realize stable charge for stabilization.

Further, the method for preparing the insoluble pectin-adriamycin conjugate into the suspension comprises adding 500 - 900mg pectin-adriamycin conjugate, 3000 - 6000mg PVP K-30 and 500 - 900mg poloxamer 188 to 100mL sterile water for injection to obtain a mixture, grinding the mixture into the suspension, and preparing the suspension in the nanosuspension.

Specifically, the following mixture ratio is preferable:
PAC (main drug): 776mg (solvent in the formula is 100mL sterile water for injection, based on adriamycin equivalent of 2mg/mL, 200mg adriamycin is required, drug loading rate of the PAC is 25.8%, 200/25.8% = 775.19mg, and rounding up to 776mg);
PVP K-30: 4000mg (w/v, 4%);
Poloxamer 188: 700mg (w/v, 0.7%);
Sterile water for injection (solvent): 100mL.

That is, 500 - 900mg pectin-adriamycin conjugate, 3000 - 6000mg PVP K-30 and 500 - 900mg poloxamer 188 are added to 100mL sterile water for injection to obtain a mixture, and the mixture is ground into the suspension, and the suspension is prepared into the nanosuspension if required.

The nanosuspension of the invention is prepared by referring to the following method based on the formula:
A preparing PVP K-30 into PVP K-30 solution with sterile water for injection;
B dry grinding and evenly mixing PAC and poloxamer 188 (the purpose of grinding is to obtain small and even particle size of the ground PAC), and adding a little PVP K-30 solution for full grinding (the purpose of full grinding is to obtain smaller and evener particle size of the PAC suspension so as to prevent blocking homogenizing valves of the high-pressure homogenizer, with preliminary particle size of the PAC suspension less than 80µm);
C adding remaining PVP K-30 solution in several times for grinding (the purpose of such operation is to clean up PAC attached to a mortar so as to prevent heavy loss of raw materials);
D performing ultrasonic treatment on the ground suspension for full mixing so as to prepare the suspension; and
E treating the suspension evenly mixed in the step D in a high-pressure homogenizer (EmulsiFlex C-3 high-pressure homogenizer, Avestin Inc., Canada); controlling pressure of the homogenizer at 4000 - 8000PSI (the purpose of such operation is to provide the homogenizer with an adaptation process so as to prevent occurrence of blockage, preferably 5000PSI) for cycle treatment of samples for 3 - 10min (the purpose of such operation is to provide the homogenizer with an adaptation process so as to prevent occurrence of blockage, preferably 3min), and then keeping the pressure of the homogenizer at 20000 - 30000PSI (the upper pressure limit of homogenizers currently available on the market is generally 30000PSI, i.e. 200Mpa, preferably 25000PSI) for cycle treatment of the samples for 15 - 30min (in the range, the longer the treatment time is, the better the PDI homogeneity value of nano particles of the nanosuspension is, and the treatment time is 20min for sake of cost factor) to obtain the PAC nanosuspension.

It shall be noted that in order to prevent rising temperature of the homogenizer during treatment at high pressure from affecting physicochemical properties of the drugs, temperature of a constant heat exchanger homogenizer used is 20 - 40°C (preferably 25°C) in the whole treatment. Adriamycin is photosensitive, thus all operations are performed away from light.

Specifically, the following method can be used for preparation in the laboratory:
A precisely weighing PVP K-30 according to the formulated amount, and preparing solution with formulated amount of sterile water for injection;
B precisely weighing and placing PAC and poloxamer 188 in an agate mortar, dry grinding for even mixing, and adding a little PVP K-30 solution for full grinding, with particle size less than 80µm (tests show that the requirement can be met by the mortar within 20min);
C adding remaining PVP K-30 solution in several times for grinding, transferring the suspension to a conical flask, cleaning up suspension attached to the mortar with a little PVP K-30 solution, merging the liquid, and performing ultrasonic treatment for full mixing (5min is ok generally); and
D Treating pretreated samples (equivalent to adriamycin equivalent of 2mg/mL) in a high-pressure homogenizer: controlling pressure of the homogenizer at 4000 - 8000PSI (preferably 5000PSI) for cycle treatment of the samples for 3 - 10min (preferably 3min), then keeping the pressure of the homogenizer at 20000 - 30000PSI (preferably 25000PSI) for cycle treatment of the samples for 15 - 30min (preferably 20min), and controlling the temperature at 20 - 40°C (preferably 25°C).

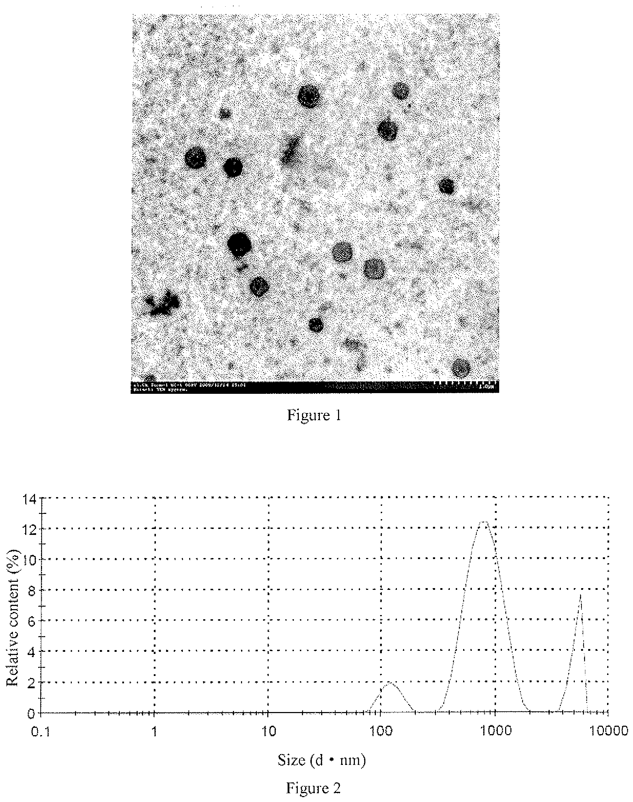

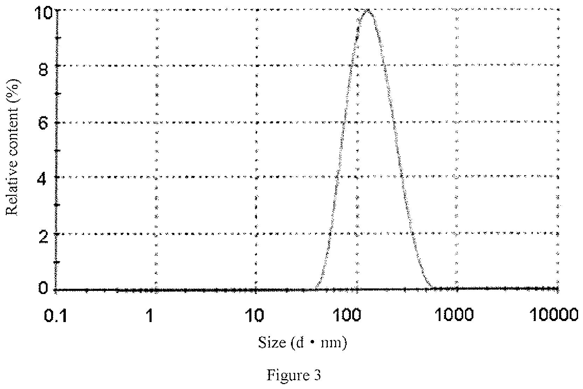

Tests show that the average particle size of the nanosuspension prepared by the method is 167.3nm, PDI polydispersity value is 0.141 (see Figure 3), and Zeta potential is -17.5mV. The result shows that preparation of the PAC nanosuspension by a high pressure homogenization method can meet various quality control indexes, and conform to requirements for particle size of the nanosuspension for injection with smaller particle size. The particle size distribution also meets requirements of the EPR effect for particle size (the particle size is required to be less than 200nm in the EPR effect), so that the PAC nanosuspension can maximize tumor targeting of the EPR; PDI value is less than 0.2, which indicates that the particle size distribution range is narrow, and proportion of large particles in the suspension can be ensured; and Zeta potential is -17.5mV, which indicates that the potential stability is higher. Particle shape of the nanosuspension is observed under a transmission electron microscopy (TEM). The TEM can help clearly observe that the particle shape of the PAC nanosuspension is of regular spherical-like shape (see Figure 1), and particle size is approximate 200nm, which is substantially consistent with results measured with a laser nano particle size analyzer.

As particles of the nanosuspension easily aggregate due to higher surface free energy, long-term stability of the PAC nanosuspension is determined as follows:
separately filling 2mg/mL freshly prepared PAC nanosuspension in 6 penicillin bottles respectively, keeping away from light, placing the penicillin bottles in a constant temperature & humidity chamber, and controlling the temperature at 25°C and humidity at 60% ± 10%; and sampling a bottle at time intervals of 3 days, 10 days, 25 days, 50 days, 90 days and 6 months respectively to determine particle size and polydispersity. Stability test results of the nanosuspension show that particles of the nanosuspension in a suspension state begin to aggregate and the particle size increases from 167.3nm to approximate 458nm after 50 days; and after 6 months, the average particle size increases to 895nm, particles of the nanosuspension seriously aggregate, nano particles and micron particles coexist (see Figure 2), serious tailing peak occurs to micron particles in determination of the particle size, and polydispersity becomes very poor (PDI value is 0.602). The phenomenon shows that although large amount of stabilizers is added to the nanosuspension, the long-term stability is still unsatisfactory. Therefore, as the particle size of nano particles is unstable, the PAC nanosuspension can not be stored for a long time in a suspension state.

The nanosuspension of the invention can be also prepared by the following method:
1. Adding 0.3 - 0.6g PAC to 0.4 - 1.6g PVP, 2 - 6ml glycerol and 40 - 60ml 1 - 3% lecithin solution as a solvent to prepare the suspension, and preparing the suspension into the nanosuspension using a high-pressure homogenizer.
   Specifically, 0.468g macromolecular insoluble pectin-adriamycin conjugate is added with 1g PVP, 3ml glycerol and 50ml 2% lecithin solution as a solvent, and ground to prepare the suspension, and treated in an ultra-high pressure nano homogenizer (T-200D homogenizer manufactured by Hebei Langfang General Machinery Manufacturing Co., Ltd.). The suspension is treated in the ultra-high pressure nano homogenizer for 3 times at 120mpa for the first time, 180mpa for the second time and 190mpa for the third time.
2. Adding 0.3 - 0.6g PAC to a mixed solvent of 0.4 - 1.6g PVP, 40 - 60ml water and DMSO to prepare the suspension, with water:DMSO = 0.5 - 0.85:0.15 - 0.5, and preparing the suspension into the nanosuspension using the high-pressure homogenizer.

Specifically, 0.468g macromolecular insoluble pectin-adriamycin conjugate is added with a mixed solvent of 1g PVP, 50ml water and DMSO (water:DMSO = 0.75:0.25) to prepare the suspension, or further the suspension is prepared into the nanosuspension using the high-pressure homogenizer. Specifically, the high pressure homogenizer is used for treatment to prepare the nanosuspension at pressure not more than 200mpa each time, (The inventor used T-200D homogenizer manufactured by Hebei Langfang General Machinery Manufacturing Co., Ltd.), specifically, the suspension can be treated in the ultra-high pressure nano homogenizer for 3 times at 120mpa for the first time, 180mpa for the second time and 190mpa for the third time.

The particle size of the nanosuspension prepared by the method is below 200nm, PDI value is less than 0.25, and absolute potential value is greater than 10mV. The insoluble drug PAC can be prepared into the nanosuspension by the high pressure homogenization method; and quality test results show that the particle size and polydispersity of nano particles of the nanosuspension meet requirements, and Zeta potential value is large, thus the suspension has higher potential stability, and the particle shape is of regular spherical-like shape observed under the transmission electron microscopy.

Stability main includes physical stability and chemical stability. The physical stability refers to spatial stability of nano particles of the PAC nanosuspension, can be directly characterized by changes of particle size and polydispersity index over time, and can be indirectly illustrated by Zeta potential of a system; and the chemical stability refers to degradation degree of effective drug concentration of the suspension over time. Defect of poor long-term stability of the PAC suspension or nanosuspension thereof is due to poor physical stability of the system.

In order to solve the long-term stability defect of the PAC nanosuspension, the inventor tried the following methods:
(1) Adding large amount of PVP K-30 to increase viscosity of the system, and prevent aggregation of nano particles: PVP K-30 served as a suspending agent to increase the viscosity of the dispersion system. According to stock equation (V = 2r²(ρ1-ρ2)g/9η, η-viscosity of a dispersion medium), the larger the viscosity of the system is, the slower the precipitation of the drug particles is, and the better the stability is. The inventor tried to add 8% PVP K-30 in the tests to increase the viscosity of the system, however, with significant increase of the viscosity of the system, pressure on the high-pressure homogenizer during preparation had to be increased, thus aggravating wear of the homogenizer. Even so, the particle size of the finally prepared PAC suspension did not produce good result, the average particle size was larger than 300nm, and the PDI value was larger than 0.25, thus the method is infeasible.
(2) Adding ionic surfactants such as sodium dodecyl sulfate (SDS) to provide charge stabilization effect, and adding large amount of SDS to the PAC nanosuspension to allow absolute Zeta potential value of the system to be larger than 30Mv. However, the stability test results were not satisfactory, the particle size and the PDI value of the suspension system increased greatly 6 months later, and even obvious precipitation, thus the method is also infeasible.

The second method can not ensure stability of the formulation after long-term storage, thus the method is inadvisable; in the first method, the addition of large amount of PVP increases production cost, and the particle size and the PDI value of the prepared nanosuspension are not optimal.

Finally, the inventor proposed to prepare the nanosuspension into a lyophilized formulation after comprehensively considering all factors, and finally verified that appearance, resolubility, particle size, etc. of the PAC nanosuspension lyophilized product meet requirements.

Lyophilized products shall be added with lyophilized support agents generally, mannitol, dextran, lactose, sucrose, glucose, sorbitol, sodium chloride and various substances can be used as lyophilized support agents, and using amount of the lyophilized support agents are different and not unified depending on different drugs and formulae, and vary widely from milligram to gram. While the substances can be used as lyophilized support agents, different lyophilized support agents have significant impact on appearance, texture, resolubility, changes in particle size of nanosuspension before and after lyophilization, etc. of lyophilized products, therefore, the inventor selected lyophilized support agents capable of providing lyophilized products with loose texture, saturated appearance, good resolubility and no major difference in particle size before and after lyophilization based on appearance, resolubility and difference in particle size before and after lyophilization as indexes. See Table 1 for detailed appearance, redissolution speed, changes in particle size before and after redissolution, etc.

**Table 1 Screening of various lyophilized support agents**

| Lyophilized support agent | Difference in particle size before and after redissolution | Appearance | Redissolution speed |
|---|---|---|---|
| Mannitol | 75 - 150nm | Loosely saturated | Fast |
| Dextran | >100nm | Loosely cavernous | Faster |
| Lactose | >100nm | Loosely cavernous | Faster |
| Sucrose | <50nm | Loosely saturated | Fast |
| Glucose | >200nm | Shrinking | Slow |
| Sorbitol | <50nm | Loosely cavernous | Faster |
| Sodium chloride | 75 - 150nm | Loosely saturated | Fast |

It can be seen from screening tests that among various lyophilized support agents, the sucrose can provide the best lyophilization effect and optimal appearance, redissolution speed, difference in particle size before and after redissolution, etc., thus the sucrose is intended to be selected as the lyophilized support agent for the nanosuspension.

Meanwhile, the inventor investigated preparation of the PAC nanosuspension into the lyophilized formulation in 6 conditions respectively, i.e. adding lactose, sucrose, glucose, sorbitol, mannitol and not adding any support agent, and the addition amount of 5 support agents were respectively determined as 40mg/mL, 80mg/mL and 120mg/mL.Test results show that in the 6 conditions, products added with 80mg/mL sucrose are the optimal, and have good appearance and resolubility and almost no difference before and after lyophilization. Therefore, 80mg/mL sucrose is determined as the preferable lyophilized support agent in the formula.

Specifically, the formula of the lyophilized formulation of the invention is as follows: the lyophilized formulation of the invention is prepared by adding 500 - 900mg PAC, 3000 - 6000mg PVP K-30, 500 - 900mg poloxamer 188 and 4000 - 12000mg sucrose to 100mL sterile water for injection before lyophilization, and preferably by adding 776mg PAC, 4000mg PVP K-30, 700mg poloxamer 188 and 8000mg sucrose to 100mL sterile water for injection before lyophilization. The specific preparation process comprises the following steps: preparing the PAC nanosuspension by the method, and then adding 40 - 120mg/mL (preferably 80mg/mL) lyophilized support agent (sucrose) to the PAC nanosuspension for full ultrasonic dissolution. In order to ensure proper lyophilization of the product, liquid height shall be generally controlled within 2cm. During lyophilization, the nanosuspension to be lyophilized is placed in a -40°C refrigerator for refreezing for 6h, and then placed in a lyophilizer (Thermo Modulyo(savanf), Thermo Scientific Corp. USA) for lyophilization for 24h.

The following suspension can be also prepared into the lyophilized formulation, that is, 0.468g PAC is added with 1g PVP, 3ml glycerol and 50ml 2% lecithin solution as a solvent, ground to prepare the suspension (the suspension can be prepared by adding 0.3 - 0.6gPAC with 0.4 - 1.6g PVP, 2 - 6ml glycerol and 40 - 60ml 1 - 3% lecithin solution as a solvent), and treated in an ultra-high pressure nano homogenizer (T-200D homogenizer manufactured by Hebei Langfang General Machinery Manufacturing Co., Ltd.). The suspension is treated in the ultra-high pressure nano homogenizer for 3 times at pressure not more than 200mpa each time (120mpa for the first time, 180mpa for the second time and 190mpa for the third time), and the prepared suspension is added with 40 - 120mg/mL lactose, sucrose, glucose, sorbitol and mannitol to prepare the lyophilized formulation according to the lyophilization process, and sucrose is preferably used as the lyophilized support agent, with preferable addition amount of 80mg/mL.

Also, 0.468g PAC can be added with a mixed solvent of 1g PVP, 50ml water and DMSO (water:DMSO = 0.75:0.25) to prepare the suspension (0.3 - 0.6g PAC can be added with a mixed solvent of 0.4 - 1.6g PVP, 40 - 60ml water and DMSO (water:DMSO = 0.5 - 0.85:0.15 - 0.5) to prepare a grinding agent so as to prepare the suspension), and treated in an ultra-high pressure nano homogenizer (T-200D homogenizer manufactured by Hebei Langfang General Machinery Manufacturing Co., Ltd.). The suspension is treated in the ultra-high pressure nano homogenizer for 3 times at 120mpa for the first time, 180mpa for the second time and 190mpa for the third time, and the prepared suspension is added with 40 - 120mg/mL lactose, sucrose, glucose, sorbitol and mannitol to prepare the lyophilized formulation according to the lyophilization process, and sucrose is preferably used as the lyophilized support agent, with preferable addition amount of 80mg/mL.

In order to investigate long-term stability of lyophilized powder injection, the following test was performed: sampling 6 bottles of the lyophilized nanosuspension (the lyophilized suspension was prepared based on a preferable lyophilized formulation formula: that is, adding 776mg PAC, 4000mg PVP K-30, 700mg poloxamer 188 and 8000mg sucrose to 100mL sterile water for injection), keeping away from light, placing the bottles in a constant temperature & humidity chamber, and controlling the temperature at 25°C and humidity at 60%±10%, and sampling a bottle at time intervals of 3 days, 10 days, 25 days, 50 days, 90 days and 6 months respectively to determine particle size and polydispersity. In the same test conditions, the particle size of the PAC nanosuspension lyophilized product was 182.3nm determined after storage for half a year, and compared with 172.6nm of freshly prepared suspension lyophilized product, the particle size almost has no major change. The test results show that the stability of the lyophilized product prepared from the suspension is greatly improved compared with that in a suspension storage state. Based on stability of the particle size of the nanosuspension, preparation of the PAC nanosuspension into the lyophilized product can ensure long-term storage stability.

Proposed administration route of the PAC nanosuspension of the invention is intravenous injection, thus the particle size shall be controlled strictly. The 6-month stability test results show that the average initial particle size of the PAC nanosuspension is 167.3nm, and the average particle size is 895nm 6 months later; particles seriously aggregate, and nano particles and micron particles coexist. The average initial particle size of the lyophilized PAC nanosuspension is 172.6nm, the average particle size is 182.3nm 6 months later, and the particle size almost has no major change. The result shows that the PAC nanosuspension can not be stored for a long time in a suspension state, and the lyophilized product has good long-term stability and can be stored for a long time. The proposed administration route of the PAC nanosuspension is intravenous injection, thus the particle size shall be controlled strictly. Compared common nanosuspensions with the lyophilized nanosuspension, the latter has absolute advantage in ensuring stability of the particle size.

The inventor also monitored the drug loading rate of the PAC formulation in long-term storage using a method as follows:

### 1. Method

### 1.1 Method for determination of drug loading rate

### 1.1.1 Preparation of standard curve of adriamycin hydrochloride

10.7mg adriamycin hydrochloride was precisely weighed and placed in a 100ml volumetric flask, diluted with sterile water for injection to volume as stock solution, and underwent ultrasonic treatment for 5min for even mixing; 0.2ml, 0.4ml, 0.8ml, 1.2ml, 1.6ml and 2ml adriamycin hydrochloride stock solution were pipetted and placed in 5ml volumetric flasks respectively, diluted with sterile water for injection to volume, and shook up. Using ultraviolet spectrophotometry, absorbance A values of adriamycin solution were determined at the series of concentration gradients at 480nm, and linear regression was performed by taking absorbance A as a vertical coordinate and mass concentration C (mg·mL-1) of adriamycin as horizontal coordinate to obtain an equation: A = 0.0178C + 0.0284, R2 = 0.9994 (n = 3).

### 1.1.2 Determination of drug loading rate of PAC nanosuspension

1mL PAC nanosuspension (prepared according to a preferable solution) was precisely pipetted and placed in a 50mL volumetric flask (concentration of the PAC nanosuspension was 7.76mg/mL), diluted with sterile water for injection to volume and shook up, and then proper amount was pipetted to determine absorbance at 480nm so as to obtain adriamycin concentration Cx with the standard curve. Drug loading rate of the PAC = (Cx/7.76)*100%.

### 1.1.3 Determination of drug loading rate of PAC lyophilized formulation

One bottle of PAC lyophilized formulation (prepared according to a preferable solution) was sampled, and redissolved in 2mL sterile water for injection into a uniform nanosuspension, 1mL redissolved PAC nanosuspension was precisely pipetted and placed in a 50mL volumetric flask (concentration of the PAC nanosuspension was 7.76mg/mL), diluted with sterile water for injection to volume and shook up, and then proper amount was pipetted to determine absorbance at 480nm so as to obtain adriamycin concentration Cx with the standard curve. Drug loading rate of the PAC = (Cx/7.76)*100%.

### 1.2 Monitoring of drug loading rate of PAC nanosuspension

2mg/mL freshly prepared PAC nanosuspension was filled in 6 penicillin bottles respectively, kept away from light, placed in a constant temperature & humidity chamber, and the temperature was controlled at 25°C, and humidity was controlled at 60% ± 10%; and a bottle was sampled at time intervals of 3 days, 10 days, 25 days, 50 days, 90 days and 6 months respectively to determine the drug loading rate according to 1.1.2.

### 1.3 Monitoring of drug loading rate of PAC lyophilized formulation

Six bottles of lyophilized PAC nanosuspension (see preparation of lyophilized formulation) were sampled, kept away from light, placed in a constant temperature & humidity chamber, and the temperature was controlled at 25°C, and humidity was controlled at 60% ± 10%; and a bottle was sampled at time intervals of 3 days, 10 days, 25 days, 50 days, 90 days and 6 months respectively to determine the drug loading rate according to 1.1.3.

### 2. Results

The drug loading rate of bulk drugs for preparation of the PAC nanosuspension was 25.8%. The drug loading rate of the PAC nanosuspension tended to decreases gradually during placement, and decreased to 20.3% 6 months later. Compared with unlyophilized nanosuspension, the drug loading rate of the PAC lyophilized formulation tended to stabilize during placement, and was 24.6% 6 months later.

PAC prepared by reacting adriamycin (ADM) with pectin is a macromolecular prodrug. Prodrug is an inactive medical precursor, has no or very low activity before being delivered to a target site, is activated under the catalysis of enzyme or non-enzyme action at the target site, and releases active substances, thus exerting pharmacological actions. PAC slowly releases ADM during storage, which has significant impact on in vivo passive targeting anticancer activity. Therefore, compared with the PAC nanosuspension, the nanosuspension lyophilized formulation has obvious advantages.

### 3. Conclusion

Taking stability of the drug loading rate of the bulk drugs into consideration, the particle size of nano particles of the nanosuspension is instable during placement, and nano particles tend to further aggregate; the stability of nano particle size of the lyophilized product prepared from the nanosuspension is enhanced; the drug loading rate of the PAC is instable in the nanosuspension with water as a medium, and decreases from 25.8% to 21.6%; and stability of the drug loading rate of the prepared lyophilized product is enhanced. Therefore, the lyophilized product prepared from the PAC nanosuspension can ensure long-term storage stability of the PAC in a better manner.

## Claims

1. A lyophilized formulation of pectin-adriamycin conjugate, **characterized in that** an insoluble pectin-adriamycin conjugate is prepared into a suspension or nanosuspension, and a lyophilized support agent is added to the suspension for lyophilization treatment to prepare the lyophilized formulation;
wherein the lyophilized support agent is sucrose.

2. The lyophilized formulation of pectin-adriamycin conjugate according to claim 1, **characterized in that** addition amount of the lyophilized support agent is 40 - 120mg/mL.

3. The lyophilized formulation of pectin-adriamycin conjugate according to claim 2, **characterized in that** the addition amount of the lyophilized support agent is 80mg/mL.

4. A preparation method of the lyophilized formulation of pectin-adriamycin conjugate according to any of claims 1 to 3, **characterized by** comprising:
(1) preparing an insoluble pectin-adriamycin conjugate into a suspension or nanosuspension; and
(2) adding a lyophilized support agent for lyophilization treatment;
wherein, a method of the lyophilization treatment comprises the following steps:
A separately filling the suspension in penicillin bottles for prefreezing at -40°C--80°C for 4 - 8h; and
B placing the penicillin bottles in a lyophilizer for lyophilization.

5. The preparation method of the lyophilized formulation of pectin-adriamycin conjugate according to claim 4, **characterized in that** the method for preparing the insoluble pectin-adriamycin conjugate into the suspension comprises adding 500 - 900mg pectin-adriamycin conjugate and a stabilizer to 100mL sterile water for injection to obtain a mixture, and grinding the mixture into the suspension.

6. The preparation method of the lyophilized formulation of pectin-adriamycin conjugate according to claim 5, **characterized in that** a nanosuspension is prepared after grinding the mixture into a suspension.

7. The preparation method of the lyophilized formulation of pectin-adriamycin conjugate according to claim 5, **characterized in that** the stabilizer is at least one of PVP, poloxamer, sodium dodecyl sulfate, polysorbate or hydroxypropyl methyl cellulose.

8. The preparation method of the lyophilized formulation of pectin-adriamycin conjugate according to claim 4, **characterized in that** the stabilizer is PVP K-30 and poloxamer 188, the method for preparing the insoluble pectin-adriamycin conjugate into the suspension comprises adding 500 - 900mg pectin-adriamycin conjugate, 3000 - 6000mg PVP K-30 and 500 - 900mg poloxamer 188 to 100mL sterile water for injection to obtain a mixture, and grinding the mixture into the suspension.

9. The preparation method of the lyophilized formulation of pectin-adriamycin conjugate according to claim 8, **characterized in that** the lyophilized formulation is prepared by adding 500 - 900mg PAC, 3000 - 6000mg PVP K-30, 500 - 900mg poloxamer 188 and 4000 - 12000mg sucrose to 100mL sterile water for injection before lyophilization.

10. The preparation method of the lyophilized formulation of pectin-adriamycin conjugate according to claim 4, **characterized in that** the method for preparing the insoluble pectin-adriamycin conjugate into the suspension comprises adding 0.4 - 1.6g PVP, 2 - 6ml glycerol and 40 - 60ml 1 - 3% lecithin solution as a solvent to 0.3 - 0.6g PAC to prepare the suspension.

11. The preparation method of the lyophilized formulation of pectin-adriamycin conjugate according to claim 4, **characterized in that** the method for preparing the insoluble pectin-adriamycin conjugate into the suspension comprises adding a mixed solvent of 0.4 - 1.6g PVP, 40 - 60ml water and DMSO to 0.3 - 0.6g PAC to prepare the suspension, wherein water:DMSO equals 0.5 - 0.85:0.15 - 0.5.

12. The preparation method of the lyophilized formulation of pectin-adriamycin conjugate according to any of claims 10 or 11, **characterized in that** a high-pressure homogenizer is used for treatment of the prepared suspension to prepare the nanosuspension.

13. The preparation method of the lyophilized formulation of pectin-adriamycin conjugate according to claim 8 , **characterized in that** all components are weighed according to claim 8 to prepare the nanosuspension of the pectin-adriamycin conjugate according to the following methods:
A preparing PVP K-30 into PVP K-30 solution with sterile water for injection;
B dry grinding and evenly mixing PAC and poloxamer 188, and adding a little PVP K-30 solution for full grinding;
C adding remaining PVP K-30 solution in several times for grinding;
D performing ultrasonic treatment on the ground suspension for full mixing so as to prepare the suspension; and
E treating the suspension evenly mixed in the step D in a high-pressure homogenizer: controlling pressure of the homogenizer at 4000 - 8000PSI for cycle treatment of samples for 3 - 10min, and then keeping the pressure of the homogenizer at 20000 - 30000PSI for cycle treatment of the samples for 15 - 30min to obtain the nanosuspension.

## Patentansprüche

1. Lyophilisierte Formulierung aus einem Pectin-Adriamycin-Konjugat, **dadurch gekennzeichnet, dass** ein unlösbares Pectin-Adriamycin-Konjugat zu einer Suspension oder Nanosuspension verarbeitet wird, und wobei ein lyophilisiertes Hilfsmittel in die Suspension zur Lyophilisierungsbehandlung eingegeben wird, um die lyophilisierte Formulierung herzustellen, wobei das lyophilisiertes Hilfsmittel Saccharose ist.

2. Lyophilisierte Formulierung aus einem Pectin-Adriamycin-Konjugat nach Anspruch 1, **dadurch gekennzeichnet, dass** die Zugabemenge des lyophilisierten Hilfsmittels 40 - 120mg/mL beträgt.

3. Lyophilisierte Formulierung aus einem Pectin-Adriamycin-Konjugat nach Anspruch 2, **dadurch gekennzeichnet, dass** die Zugabemenge des lyophilisierten Hilfsmittels 80mg/mL beträgt.

4. Verfahren zur Herstellung einer lyophilisierten Formulierung aus einem Pectin-Adriamycin-Konjugat nach einem der Ansprüche 1 bis 3, **dadurch gekennzeichnet, dass** umfassend:
(1) Verarbeiten eines unlösbaren Pectin-Adriamycin-Konjugats zu einer Suspension oder Nanosuspension; und
(2) Eingeben eines lyophilisierten Hilfsmittels zur Lyophilisierungsbehandlung;
wobei ein Verfahren zur Lyophilisierungsbehandlung folgende Schritte umfasst:
A. Füllen der Suspension jeweils in jeweiligen Penicillinflaschen zum Vorfrieren bei -40°C bis -80°C für 4 bis 8 Stunden,
B. Legen der Penicillinflaschen in eine Lyophilisierungsanlage zur Lyophilisierung ein.

5. Verfahren zur Herstellung einer lyophilisierten Formulierung aus einem Pectin-Adriamycin-Konjugat nach Anspruch 4, **dadurch gekennzeichnet, dass** das Verfahren zur Verarbeitung eines unlösbaren Pectin-Adriamycin-Konjugats zu einer Suspension oder Nanosuspension das Eingeben des Pectin-Adriamycin-Konjugats von 500-900mg und eines Stabilisators in das sterile Wasser zur Injektion von 100ml umfasst, um eine Mischung zu erhalten, und Vermahlen der Mischung zur Suspension.

6. Verfahren zur Herstellung einer lyophilisierten Formulierung aus einem Pectin-Adriamycin-Konjugat nach Anspruch 5, **dadurch gekennzeichnet, dass** nach dem Vermahlen der Mischung zur Suspension eine Nanosuspension hergestellt wird.

7. Verfahren zur Herstellung einer lyophilisierten Formulierung aus einem Pectin-Adriamycin-Konjugat nach Anspruch 5, **dadurch gekennzeichnet, dass** der Stabilisator mindestens eines von PVP, Poloxamer, Natriumdodecylsulfat, Polysorbat oder Hydroxypropylmethylcellulose ist.

8. Verfahren zur Herstellung einer lyophilisierten Formulierung aus einem Pectin-Adriamycin-Konjugat nach Anspruch 4, **dadurch gekennzeichnet, dass** der Stabilisator PVP K-30 und Poloxamer 188 ist, wobei das Verfahren zur Verarbeitung eines unlösbaren Pectin-Adriamycin-Konjugats zu einer Suspension das Eingeben des Pectin-Adriamycin-Konjugats von 500-900mg, von PVP K-30 von 3000 bis 6000mg und Poloxamer 188 von 500-900mg in das sterile Wasser zur Injektion von 100ml umfasst, um eine Mischung zu erhalten, und Vermahlen der Mischung zur Suspension.

9. Verfahren zur Herstellung einer lyophilisierten Formulierung aus einem Pectin-Adriamycin-Konjugat nach Anspruch 8, **dadurch gekennzeichnet, dass** durch das Eingeben von PAC von 500-900mg, PVP K-30 von 3000 bis 6000mg, Poloxamer 188 von 500-900mg und die Saccharose von 4000 bis 12000mg in das sterile Wasser zur Injektion von 100ml vor der Lyophilisierung die lyophilisierte Formulierung hergestellt wird.

10. Verfahren zur Herstellung einer lyophilisierten Formulierung aus einem Pectin-Adriamycin-Konjugat nach Anspruch 4, **dadurch gekennzeichnet, dass** das Verfahren zur Verarbeitung eines unlösbaren Pectin-Adriamycin-Konjugats zu einer Suspension das Eingeben von PVP von 0,4-1,6g, Glycerin von 2-6ml und 1-3% Lecithinlösung von 40-60ml als Lösungsmittel in PAC von 0,3 - 0,6 g zur Herstellung der Suspension umfasst.

11. Verfahren zur Herstellung einer lyophilisierten Formulierung aus einem Pectin-Adriamycin-Konjugat nach Anspruch 4, **dadurch gekennzeichnet, dass** das Verfahren zur Verarbeitung eines unlösbaren Pectin-Adriamycin-Konjugats zu einer Suspension das Eingeben einer Mischung aus PVP von 0,4-1,6g, Wasser von 40-60ml und DMSO in PAC von 0,3 - 0,6 g zur Herstellung der Suspension umfasst, wobei Wasser:DMSO 0,5 bis 0,8: 0,15 bis 0,5 ist.

12. Verfahren zur Herstellung einer lyophilisierten Formulierung aus einem Pectin-Adriamycin-Konjugat nach Anspruch 10 oder 11, **dadurch gekennzeichnet, dass** ein Hochdruckhomogenisator für die Behandlung der hergestellten Suspension zur Herstellung der Nanosuspension verwendet wird.

13. Verfahren zur Herstellung einer lyophilisierten Formulierung aus einem Pectin-Adriamycin-Konjugat nach Anspruch 8, **dadurch gekennzeichnet, dass** alle Komponenten gemäß Anspruch 8 gewogen werden, um die Nanosuspension des Pektin-Adriamycin-Konjugats gemäß folgenden Verfahren herzustellen:
A. Herstellung von PVP K-30 zur PVP K-30 Lösung mit dem sterilen Wasser zur Injektion;
B. Trockenmahlen und gleichmäßiges Mischen von PAC und Poloxamer 188 und Eingeben einer wenigen Menge von PVP K-30-Lösung zum vollständigen Mahlen;
C. Eingeben der verbleibenden PVP K-30-Lösung in Mehrmals zum Mahlen;
D. Durchführung einer Ultraschallbehandlung der gemahlenen Suspension zum vollständigen Mischen, um die Suspension herzustellen; und
E. Behandlung der in Schritt D gleichmäßig vermischten Suspension in einem Hochdruckhomogenisator: Kontrolle des Drucks des Homogenisators bei 4000 - 8000 PSI für die zyklische Behandlung von Proben für 3 - 10 min und Halten des Drucks des Homogenisators bei 20000 - 30000 PSI für die zyklische Behandlung der Proben für 15 - 30 min, um die Nanosuspension zu erhalten.

## Revendications

1. Formulation lyophilisée de conjugué pectine-adriamycine, **caractérisée en ce qu'**un conjugué insoluble de pectine-adriamycine est préparé en suspension ou en nanosuspension et qu'un agent support lyophilisé est ajouté à la suspension pour le traitement de lyophilisation afin de préparer la formulation lyophilisée.
dans lequel l'agent de support lyophilisé est le saccharose.

2. Formulation lyophilisée de conjugué pectine-adriamycine selon la revendication 1, **caractérisée en ce que** la quantité d'addition de l'agent de support lyophilisé est de 40 à 120 mg / ml.

3. Formulation lyophilisée de conjugué pectine-adriamycine selon la revendication 2, **caractérisée en ce que** la quantité ajoutée de l'agent de support lyophilisé est de 80 mg / ml.

4. Procédé de préparation de la formulation lyophilisée de conjugué pectine-adriamycine selon l'une quelconque des revendications 1 à 3, **caractérisé en ce qu'**il comprend:
(1) préparer un conjugué insoluble de pectine-adriamycine dans une suspension ou une nanosuspension; et
(2) ajouter un agent de support lyophilisé pour un traitement de lyophilisation ;
dans lequel un procédé de traitement de lyophilisation comprend les étapes suivantes :
A remplissage séparé de la suspension dans des bouteilles de pénicilline pour une pré-congélation entre -40 °C et 80 °C pendant 4 à 8 heures ; et
B placer les bouteilles de pénicilline dans un lyophilisateur pour la lyophilisation.

5. Procédé de préparation de la formulation lyophilisée de conjugué pectine-adriamycine selon la revendication 4, **caractérisé en ce que** le procédé de préparation du conjugué insoluble pectine-adriamycine dans la suspension comprend l'ajout de 500 à 900 mg de conjugué pectine-adriamycine et un stabilisant à 100 ml d'eau stérile pour préparations injectables pour obtenir un mélange et broyage du mélange dans la suspension.

6. Procédé de préparation de la formulation lyophilisée de conjugué pectine-adriamycine selon la revendication 5, **caractérisé en ce que** une nanosuspension après broyage du mélange en suspension est préparée.

7. Procédé de préparation de la formulation lyophilisée de conjugué pectine-adriamycine selon la revendication 5, **caractérisé en ce que** le stabilisant est au moins l'un des composés PVP, poloxamère, dodécylsulfate de sodium, polysorbate et hydroxypropylméthylcellulose.

8. Procédé de préparation de la formulation lyophilisée de conjugué pectine-adriamycine selon la revendication 4, **caractérisé en ce que** le stabilisant est la PVP K-30 et le poloxamère 188, le procédé de préparation du conjugué insoluble pectine-adriamycine dans la suspension comprenant l'ajout de 500 - 900 mg de conjugué pectine-adriamycine, 3000 à 6000 mg de PVP K-30 et 500 à 900 mg de poloxamère 188 à 100 ml d'eau stérile pour préparations injectables afin d'obtenir un mélange et de broyer le mélange dans la suspension.

9. Procédé de préparation de la formulation lyophilisée de conjugué pectine-adriamycine selon la revendication 8, **caractérisé en ce que** la formulation lyophilisée est préparée en ajoutant 500 - 900 mg de PAC, 3000 - 6000 mg de PVP K-30, 500 - 900 mg de poloxamer 188 et 4000 - 12 000 mg de saccharose à 100 ml d'eau stérile pour injection avant lyophilisation.

10. Procédé de préparation de la formulation lyophilisée de conjugué pectine-adriamycine selon la revendication 4, **caractérisé en ce que** le procédé de préparation du conjugué insoluble pectine-adriamycine dans la suspension comprend l'addition de 0,4 à 1,6 g de PVP, 2 à 6 ml de glycérol et 40 - 60 ml de solution de lécithine à 1 - 3% en tant que solvant pour 0,3 à 0,6 g de PAC pour préparer la suspension.

11. Procédé de préparation de la formulation lyophilisée de conjugué pectine-adriamycine selon la revendication 4, **caractérisé en ce que** le procédé de préparation du conjugué insoluble pectine-adriamycine dans la suspension comprend l'addition d'un solvant mélangé de 0,4 à 1,6 g de PVP, 40 à 60 ml d'eau et DMSO à 0,3 - 0,6 g de PAC pour préparer la suspension, où l'eau: DMSO est égal à 0,5 - 0,85: 0,15-0,5.

12. Procédé de préparation de la formulation lyophilisée de conjugué pectine-adriamycine selon l'une quelconque des revendications 10 ou 11, **caractérisé en ce qu'**un homogénéisateur à haute pression est utilisé pour le traitement de la suspension préparée afin de préparer la nanosuspension.

13. Procédé de préparation de la formulation lyophilisée de conjugué pectine-adriamycine selon la revendication 8, **caractérisé en ce que** tous les composants sont pesés selon la revendication 8 pour préparer la nanosuspension du conjugué pectine-adriamycine selon les méthodes suivantes:
A préparation de PVP K-30 dans une solution de PVP K-30 avec de l'eau stérile pour injection;
B broyage à sec et mélange homogène du PAC et du poloxamer 188 et ajout d'un peu de solution de PVP K-30 pour un broyage complet;
C ajouter la solution de PVP K-30 restante plusieurs fois pour le broyage;
D effectuer un traitement par ultrasons sur la suspension broyée pour un mélange complet afin de préparer la suspension; et
E traiter la suspension uniformément mélangée à l'étape D dans un homogénéisateur haute pression: contrôler la pression de l'homogénéisateur à 4000 - 8000 PSI pour le traitement en cycle des échantillons pendant 3 à 10 min, puis maintenir la pression de l'homogénéisateur à 20000 - 30000 PSI pour le cycle de traitement des échantillons pendant 15 à 30 minutes pour obtenir la nanosuspension.
